# EUROPEAN PATENT APPLICATION

(11) **EP 1 035 216 A1**
(43) Date of publication of application: **13.09.2000**
(21) Application number: 00301777.9
(22) Date of filing: 03.03.2000
(51) Int. Cl.: C12Q 1/68, G01N 27/02

(54) **Method of and apparatus for the detection of analytes**

(30) Priority: 05.03.1999 GB 9905148
(71) Applicant: Azur Environmental Ltd., Wokingham, Berkshire RG41 5TU (GB)
(72) Inventor: sang, Edman Dr, Dept. of Chemistry, RG6 6AD (GB); Amini, Fahim Dr, Wolkingham, Berkashire, RG41 5TU (GB); Davis, Alison Ms, Winnersh Triangle, Wokingham, Berkshire (GB)
(74) Representative: Baldock, Sharon Claire

(57) **Abstract**

The invention provides a method of detecting or quantitatively measuring analytes, particularly nucleic acids, using a semiconductor electrode surface coated with a rare earth metal oxide. Apparatus for use in the method is also provided.

## Description

The present invention is concerned with apparatus and methods for use in the detection of analytes, particularly nucleic acids. In particular, the invention is concerned with the use of rare earth metal oxide semiconductor materials in the detection of nucleic acid hybridization.

Many of the currently available systems for the detection of small amounts of nucleic acids are based on DNA/RNA amplification techniques, such as the polymerase chain reaction (PCR), transcription mediated amplification (TMA) and strand displacement amplification (SDA), which are used to amplify minuscule amounts of nucleic acid into detectable quantities. The amplified product is then detected using fluorescent or chemiluminescent signal detection. Although these systems are extremely sensitive and can be used quantitatively, they are time consuming, require a very clean starting sample, expensive equipment and reagents and very often must be performed by a skilled operator.

Recently, alternative methods of nucleic acid detection based on DNA chip technology have been developed for use in applications such as gene discovery, gene expression studies and mutation/polymorphism detection (for a review of this technology see Ramsay, Nature Biotechnology, 16: 40-44). In general, DNA chip techniques involve the immobilisation of DNA on a solid surface such as glass. Two variant detection formats can be used; in the first format sample DNA immobilized on the solid support is exposed to a set of labelled probes, either separately or as a mixture, in the second format an array of oligonucleotide probes immobilized on the solid support is exposed to labelled sample DNA.

Hybridization between sample DNA and probes is most commonly detected using a fluorescent readout but other techniques such as autoradiography and mass spectrometry have also been employed for this purpose. However, all of these detection techniques have disadvantages in that they require expensive reagents and/or specialised instrumentation. Fluorescent and chemiluminescent detection methods in particular require the use of expensive labelled nucleic acid probes. Attention has therefore focussed on the development of alternative detection methodologies that do not suffer from these disadvantages.

International patent application number WO 98/57159 describes a method for use in the direct detection of nucleic acids based on hybridization and electron transfer. This method is based upon the use of a probe labelled with a covalently bound ferrocenyl group which can hybridize to target nucleic acid molecules and is indirectly connected to an electrode surface via phenyl acetylene molecular 'wires'. Target DNA-labelled probe complexes are detected via electron transfer between the redox active ferrocenyl group and the electrode.

An alternative method for the direct detection of nucleic acid hybridization was described by Lawrence *et al*. (abstract presented at the 5th World Congress on Biosensors, Berlin 3rd-5th June 1998). This method uses a silicon semiconductor/SiO₂ insulator/electrolyte structure as a sensor for DNA hybridization. Single stranded oligonucleotide probes are anchored to the SiO₂ insulator surface via a silane layer. Hybridization between the probes and a target DNA sequence modifies the surface charge at the electrolyte/SiO₂ interface which in turn induces a change in the flat band potential in the semiconductor via a field effect.

The present inventors have developed an alternative method for the direct detection of nucleic acids based on the surprising observation that coating of a rare earth metal oxide semiconductor material with large biomolecules such as DNA results in detectable changes in the AC impedance of the semiconductor material. As will be discussed below, the use of rare earth metal oxides in this method confers various advantages over the previously known direct detection methods.

Accordingly, in a first aspect the invention provides a method of detecting or quantitatively measuring an analyte in a sample, which method comprises the steps of;
(a) providing a sensing electrode comprising a semiconductor electrode surface, which semiconductor electrode surface comprises a rare earth metal oxide;
(b) coating the semiconductor electrode surface with a layer of probe molecules capable of binding to the target analyte;
(c) placing the sensing electrode in contact with the sample to be tested; and
(d) measuring the change in AC impedance of the sensing electrode.

In a further aspect the invention provides a method of detecting or quantitatively measuring an analyte in a sample, which method comprises the steps of;
(a) providing a sensing electrode comprising a semiconductor electrode surface comprising a rare earth metal oxide, which semiconductor electrode surface is coated with a layer of probe molecules capable of binding to the target analyte;
(b) placing the sensing electrode in contact with the sample; and
(c) measuring the change in AC impedance of the sensing electrode.

The methods of the invention employ a sensing electrode comprising semiconductor electrode surface comprising a rare earth metal oxide. By the term 'semiconductor electrode surface' it is to be understood that an area of the surface of the sensing electrode is composed of a semiconductor material comprising a rare earth metal oxide. In a preferred embodiment the semiconductor material is a lanthanide oxide based material. Suitable lanthanide oxides include the oxides of Cerium, Praseodymium, Neodymium, Samarium, Europium, Gadolinium, Terbium, Dysprosium, Holmium, Erbium and Ytterbium. The oxides of Cerium and Praseodymium, especially Pr₆O₁₁ which contains a mixture of Pr³⁺ and Pr⁴⁺, are particularly suitable for use in the invention because their intrinsic conductivities are very high. In addition to the lanthanide oxides, semiconductor oxides of Thorium, Zirconium, Yttrium and Tin are also suitable for use in the invention. Although these are not strictly classed as rare earth metals, for the purposes of this application semiconducting oxides of these elements are to be included within the scope of the term 'rare earth metal oxides' as used herein.

It has been previously reported that changes in the electrical properties of rare earth metal oxides occur in response to the presence of ethanol (see Tsang and Bulpitt (1998), Sensor and Actuators B: Chemical, **52**: pp226-235). When rare earth metal oxide materials are heated in air oxygen molecules from the air adsorb onto the semiconductor material surface, withdrawing electrons from the bulk to form negatively charged oxygen species and depleting each oxide particle of electrons. Ethanol in the air reacts with the negatively charged oxygen species causing the re-injection of electrons back into the electron-depleted region. This is detectable as a change in DC resistance of the semiconductor material.

The present inventors have surprisingly found that a detectable reduction in AC impedance occurs when a rare earth oxide semiconductor material is coated with large biomolecules such as DNA. This effect is not the result of any chemical reaction between the DNA and reactive oxygen species at the semiconductor surface, rather it is based upon a change in both the capacitance and resistance components of the AC impedance and therefore reflects a change in the charge (electron) distribution at the interface of the adsorbed nucleic acid with the rare earth oxide based semiconducting electrode. Hence, the conductivity of the semiconductor oxide is altered by the adsorbed nucleic acid materials.

The use of lanthanide oxides in the methods of the invention confers various advantages over the previously described methods of direct detection. Lanthanide oxides are generally more conductive than silicon. In other words the *E*_{*q*} values of lanthanide oxides such as Pr₆O₁₁ are smaller than that of silicon. *E*_{*q*} is the energy gap between the highest occupied energy level (the valence band) and the lowest unoccupied energy level (the conduction band). It follows that because of the smaller *E*_{*g*}, less sensitive and therefore less expensive impedance analysis equipment is required for use with detectors based on lanthanide oxides such as Pr₆O₁₁ than are required for use with silicon based detectors. A second advantage of the smaller *E*_{*g*} of lanthanide oxides compared to silicon is that the whole detection method can be carried out at lower temperatures which favour nucleic acid hybridization and will be less damaging to any additional components of the detection system (e.g. any amplification enzymes used to amplify target nucleic acids).

In a further embodiment the semiconductor properties of the rare earth metal oxide may be altered by doping. Doping is the process of altering the properties of a semiconductor by addition of known impurities to it. The impurity may be either a donor impurity or an acceptor impurity. When a donor impurity is added the semiconductor is made more conductive as more electrons are available for excitation into the conduction band (n-type semiconductor). An acceptor impurity depletes the semiconductor of available electrons (p-type semiconductor). The lanthanide oxides are particularly amenable to doping as compared to other semiconductor materials such as silicon-based semiconductors. For example, the conductivity of lanthanide oxides can be increased by doping with inorganic promoters (Na, K, Co, Bi etc).

The method of the invention requires the semiconductor electrode surface to be coated with a layer of probe molecules. As will be illustrated in the examples given herein, one way in which this can be achieved is by placing the sensing electrode in contact with an aqueous solution containing probe molecules and allowing the probe molecules to adsorb to the semiconductor electrode surface of the sensing electrode. Surprisingly, the probe molecules are able to adsorb directly to the surface of a rare earth metal oxide semiconductor material without the need for any intermediate layer or special covalent attachment. This may be due to the strong binding (adsorption) of nucleic acid materials with the 'surface oxygen' of the rare earth oxides compared to other semiconductor materials. The adsorption interaction is non-specific, thus any type of probe molecules can be adsorbed onto the surface of the sensing electrode making the system extremely versatile. Although direct adsorption to the semiconductor surface is the simplest and therefore the preferred way of coating the sensing electrode with probe molecules, alternative coating methods such as covalent attachment could also be used for this purpose and are therefore to be included within the scope of the invention.

The probe molecules may be any type of biomolecule which is capable of specifically binding to an analyte. Preferred probe molecules are DNA, RNA, PNA, antibodies or fragments of antibodies which retain specific antigen binding activity (e.g. Fab₂ fragments).

A sensing electrode with DNA, RNA or PNA probes coated thereon may be used to detect nucleic acids capable of specifically hybridizing to the probe molecules. PNA (peptide nucleic acid) is a synthetic molecule comprising a neutral backbone of polypeptide to which are attached nucleic acid bases that allow hybridization to complementary RNA or DNA sequences. The charge-free nature of the PNA backbone results in a higher thermal stability of PNA/DNA and PNA/RNA duplexes compared to those of DNA/DNA or DNA/RNA, this stability being attributable to the lack of charge repulsion between PNA and DNA or RNA. Hence, sensors having immobilised PNA probes exhibit higher affinity for a given target DNA molecule as compared with DNA probes with identical sequence.

Coating of the semiconductor electrode surface with probe molecules, for example using non-specific adsorption, results in a first change in AC impedance of the sensing electrode. Following exposure of the sensing electrode with adsorbed probes to a sample containing analyte, specific binding of the probe molecules to target analyte results in a second change in AC impedance of the sensing electrode. Again, it is the capacitance and resistance components of the AC impedance which are observed to change. In order that the magnitude of this second change in AC impedance can be used to provide a quantifiable measurement of the amount of analyte present in a sample it is necessary to prevent any non-specific binding of the analyte directly to the semiconductor electrode surface of the sensing electrode. One way this can be accomplished is by ensuring that the semiconductor electrode surface is saturated with probe molecules before the sensing electrode is exposed to the sample containing analyte. As an alternative to saturating the semiconductor surface with probe a chemical blocking or insulating agent could be used to cover any semiconductor surface remaining exposed following coating with probe molecules.

The AC impedance of the sensing electrode is measured by assembling an electrical circuit comprising the sensing electrode and an inert reference electrode in contact with an electrolyte solution. Suitable inert reference electrodes include a platinum electrode. AC impedance is then measured using standard techniques known in the art.

In the examples given herein deionised water is used as electrolyte but other aqueous electrolyte solutions could have been used with equivalent effect. When the method is used in the detection of nucleic acids the salt concentration in the electrolyte solution may be varied in order to vary the stringency of the hybridization between the immobilised probe and target nucleic acids.

The method of the invention is useful in a wide range of applications requiring detection and/or quantitation of nucleic acids such as biological analysis, medical diagnosis and environmental monitoring. The method is particularly useful in the species-specific detection of bacterial chromosomes. It may be used in the detection of *E. coli* in drinking water and a wide range of other environmental monitoring applications. The method may also be used in the detection of single base mismatches between the probe and target sequence, allowing rapid DNA fingerprinting. The use of antibody probes allows the detection of a wide range of different analytes, including DNA, whole cells, pesticides or any other organic or inorganic molecules.

In a second aspect the invention provides an apparatus for use in the detection or quantitative measurement of analytes, which apparatus comprises an inert reference electrode and a sensing electrode comprising a semiconductor electrode surface, which semiconductor electrode surface comprises a rare earth metal oxide.

In a further aspect the invention provides a sensing electrode comprising a semiconductor electrode surface comprising a rare earth metal oxide, which semiconductor electrode surface is coated with a layer of probe molecules capable of binding to a target analyte.

As described above, the rare earth metal oxide is preferably a lanthanide oxide such as, for example, oxides of Cerium, Praseodymium, Neodymium, Samarium, Europium, Gadolinium, Terbium, Dysprosium, Holmium, Erbium and Ytterbium.

In one embodiment the sensing electrode comprises an inert electrode such as, for example, a platinum electrode coated with layer of semiconductor material comprising a rare earth metal oxide. The layer of semiconductor material may cover the whole or part of the surface of the inert electrode. In a preferred embodiment the layer of semiconductor material is provided in the form of a disc of semiconductor material which is physically adjoined to the surface of an inert electrode such that electrical connection is maintained between the semiconductor material and the inert electrode.

In an alternative embodiment the sensing electrode and inert reference electrode are provided in the form of a microchip. When the device is provided in this format an electronic addressing approach (see Cheng *et al.* (1998) Nature Biotechnology, **16:** 541-552) could be employed to direct probe molecules onto the semiconductor electrode surface of the sensing electrode.

The apparatus of the invention may optionally further comprise means for measuring the impedance of the sensing electrode.

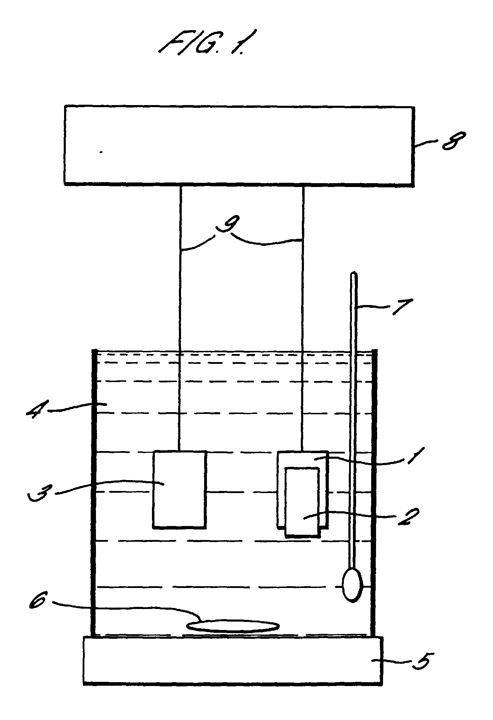

The present invention will be further understood with reference to the following Examples together with the accompanying Figures in which:-
**Figure 1** is a schematic diagram of an electrochemical cell comprising a sensing electrode (1) with a rare earth metal oxide semiconductor surface (2) and an inert reference electrode (3) immersed in electrolyte solution (4) and the whole assembly is placed on a heated stirrer plate (5) the magnetic follower (6) being placed in the electrolyte solution. Temperature is monitored using a thermometer (7). The electrodes are connected to an AC impedance analyser (Solartron 1260 Gain-Phase Analyser) (8) by means of platinum wires (9) .

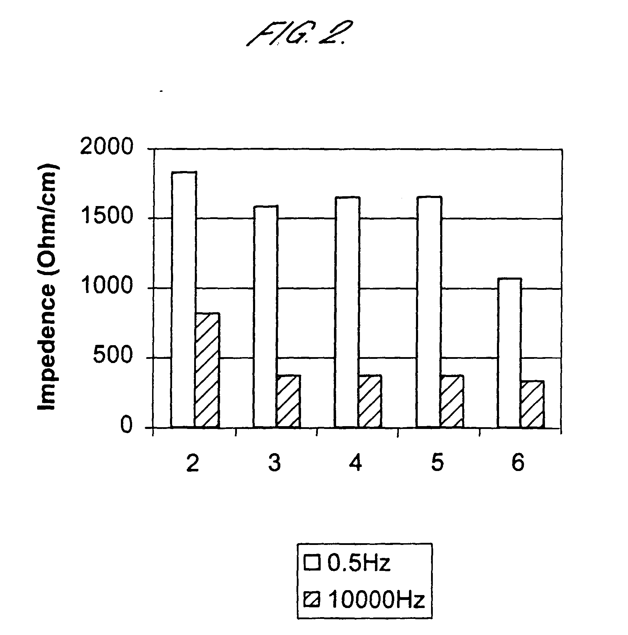

**Figure 2** is a graph to show the changes in AC impedance of a semiconductor upon adsorption of a layer of probe molecules and upon hybridization between the probe molecules and target DNA. Numbers 2 to 6 on the x-axis refer to sensing electrode/electrolyte combinations 2 to 6 listed in Table 1.

### Example 1

### (1) Assembly of an electrochemical cell.

A simple electrochemical cell was assembled comprising two platinum electrodes, positioned at a distance of approximately 1cm apart and connected to an AC impedance analyser (Solartron 1260 Gain-Phase Analyser) by platinum wires. The circuit was completed by 200mls de-ionised water acting as an electrolyte.

The de-ionised water was maintained at a constant temperature of 76°C using a heated magnetic stirrer. The working temperature was maintained constant at this temperature throughout the following steps of the procedure since the AC impedance of rare earth metal oxide semiconductors is observed to vary with temperature.

The AC impedance of the de-ionised water alone prior to the addition of semiconductor was recorded as 1.6x10⁴ Ω at a constant voltage 0.5V 0.5 Hz AC. A semiconductor electrode surface was then provided by adjoining a 1cm diameter disc of lanthanide oxide (Praseodymium oxide, Pr₆O₁₁) to the surface of one of the platinum electrodes (shown schematically in Figure 1). Following addition of the Praseodymium oxide disc the AC impedance of the electrode was recorded as 1830Ω at 0.5V 0.5 Hz AC.

### (2) Addition of probe molecules.

Single stranded oligonucleotide 30-mer probes (ECO IR primers) were added to the electrolyte solution and adsorbed to the surface of the semi-conductor. The ECO IR probes used were specific for a target in the *E. coli* chromosome. Following addition of oligonucleotides to the electrolyte solution the AC impedance of the semiconductor electrode was measured across a wide sweep of frequencies (0.5-10⁶ Hz) keeping a constant voltage of 0.5V. The AC impedance of the semiconductor was observed to decrease upon the addition of oligonucleotide (see Table 1). A total of 18.2 nmol. (167.2µg) oligonucleotide was required to saturate the surface of the disc of semiconductor, after which the addition of further oligonucleotide resulted in no further change in AC impedance.

### (3) Detection of target DNA.

To test for the specificity of the ECO IR probes *Pseudomonas aeruginosa* chromosomal DNA, which has no sequence complementarity to the ECO IR primer, was added to the electrolyte solution whilst maintaining the temperature of the electrolyte at 76°C. No quantifiable change in AC impedance of the semiconductor electrode was observed.

After addition of target *E. coli* DNA the electrolyte was left to cool from 76°C to 50°C to allow hybridization to take place. The electrolyte was then re-heated to 76°C in order to record AC impedance measurements under the same conditions. A further shift in AC impedance of the semiconductor electrode to 1070Ω was observed (summarised in table 1 and shown graphically in Figure 2). The observed decrease in AC impedance reflects hybridization between the ECO IRs probe and its complementary target in the *E. coli* genome.

**Table 1**

| | | | AC impedance (Ω/cm) | |
|---|---|---|---|---|
| | Sensing electrode | Electrolyte | 0.5Hz | 10000Hz |
| 1 | Pt (78°C) | | 16000 | 15000 |
| 2 | Pt+Pr₆O₁₁ (78°C) | | 1830 | 820 |
| 3 | Pt+Pr₆O₁₁ (76°C) | Oligo | 1587 | 370 |
| 4 | Pt+Pr₆O₁₁ (76°C) | Oligo+PA | 1651 | 373 |
| 5 | Pt+Pr₆O₁₁ (76°C) | Oligo+PA+EC | 1654 | 372 |
| 6 | Pt+Pr₆O₁₁(76°C-50°C-76°C) | Oligo+PA+EC | 1070 | 328 |
| Key: Pt-Platinum electrode; oligo-ECO IR oligonucleotide probes; PA-*Pseudomonas aeruginosa*; EC-*E. coli.* | | | | |

## Claims

1. A method of detecting or quantitatively measuring an analyte in a sample, which method comprises the steps of;
(a) providing a sensing electrode comprising a semiconductor electrode surface, which semiconductor electrode surface comprises a rare earth metal oxide;
(b) coating the semiconductor electrode surface with a layer of probe molecules capable of binding to the target analyte;
(c) placing the sensing electrode in contact with the sample; and
(d) measuring the change in AC impedance of the sensing electrode.

2. A method as claimed in claim 1 wherein the rare earth metal oxide is a lanthanide oxide, thorium oxide, tin oxide, zirconium oxide or yttrium oxide.

3. A method as claimed in claim 2 wherein the lanthanide oxide is an oxide of Cerium, Praseodymium, Neodymium, Samarium, Europium, Gadolinium, Terbium, Dysprosium, Holmium, Erbium or Ytterbium.

4. A method as claimed in any one of claims 1 to 3 wherein the probe molecules are DNA, RNA, PNA or antibodies.

5. An apparatus for use in the detection or quantitative measurement of analytes, which apparatus comprises an inert reference electrode and a sensing electrode comprising a semiconductor electrode surface, which semiconductor electrode surface comprises a rare earth metal oxide.

6. An apparatus as claimed in claim 5 wherein the rare earth metal oxide is a lanthanide oxide, thorium oxide, tin oxide, zirconium oxide or yttrium oxide.

7. An apparatus as claimed in claim 6 wherein the lanthanide oxide is an oxide of Cerium, Praseodymium, Neodymium, Samarium, Europium, Gadolinium, Terbium, Dysprosium, Holmium, Erbium or Ytterbium .

8. An apparatus as claimed in any one of claims 5 to 7 which further comprises means for measuring the AC impedance of the semiconductor electrode surface.

9. A method of detecting or quantitatively measuring an analyte in a sample, which method comprises the steps of;
(a) providing a sensing electrode comprising a semiconductor electrode surface comprising a rare earth metal oxide, which semiconductor electrode surface is coated with a layer of probe molecules capable of binding to the target analyte;
(b) placing the sensing electrode in contact with the sample; and
(c) measuring the change in AC impedance of the sensing electrode.

10. A method as claimed in claim 10 wherein the rare earth metal oxide is a lanthanide oxide, thorium oxide, tin oxide, zirconium oxide or yttrium oxide.

11. A method as claimed in claim 11 wherein the lanthanide oxide is an oxide of Cerium, Praseodymium, Neodymium, Samarium, Europium, Gadolinium, Terbium, Dysprosium, Holmium, Erbium or Ytterbium.

12. A sensing electrode comprising a semiconductor electrode surface comprising a rare earth metal oxide, which semiconductor electrode surface is coated with a layer of probe molecules capable of binding to a target analyte.

13. An electrode as claimed in claim 12 wherein the rare earth metal oxide is a lanthanide oxide, thorium oxide, tin oxide, zirconium oxide or yttrium oxide.

14. An electrode as claimed in claim 13 wherein the lanthanide oxide is an oxide of Cerium, Praseodymium, Neodymium, Samarium, Europium, Gadolinium, Terbium, Dysprosium, Holmium, Erbium or Ytterbium.

15. An electrode as claimed in any one of claims 12 to 14 wherein the probe molecules are DNA, RNA, PNA or antibodies.
